# EUROPEAN PATENT APPLICATION

(11) **EP 1 277 453 A1**
(43) Date of publication of application: **22.01.2003**
(21) Application number: 02290505.3
(22) Date of filing: 01.03.2002
(51) Int. Cl.: A61F 5/56

(54) **Device for preventing nasal obstruction**

(30) Priority: 20.07.2001 KR 2001043889
(71) Applicant: Chang Sun, Kim, Inchun-Shi (KR)
(72) Inventor: Chang Sun, Kim, Inchun-Shi (KR)
(74) Representative: Portal, Gérard

(57) **Abstract**

Disclosed is a device for preventing nasal obstruction and preventing straining of the walls of the nasal passages, by inserting it into the nasal cavities. With this configuration, straining of the walls of the nasal passages is prevented with the aid of two main support plates (4) and a plurality of expansion-support plates (5), simply by inserting two retainers (2) integrally formed on both ends of a U-shaped supporter (3) into the nasal cavities, respiration through the air passages created between the main support plates and the expansion-support plates becomes smooth, thereby preventing apnea or snoring during sleep due to nasal obstruction, having a good sleep, and preventing occurence of heart disease due to insufficient oxygen.

## Description

### BACKGROUND OF THE INVENTION

### Field of the Invention

The present invention relates in general to a device for preventing nasal obstruction, and more particularly, to a device for preventing nasal obstruction, which is inserted into nasal cavities of a person so as to prevent the openings of the nasal cavities from being obstructed; in other words, to prevent a condition that the walls of the nasal cavities are strained, the device being comprised of two retainers integrally combined with a U-shaped supporter, wherein the retainers are each formed by arranging a plurality of expansion-support plates at regular intervals so as to form air passages between the plates, the expansion-support plates of the retainers thus preventing the openings of the nasal cavities from being obstructed when the retainers are inserted into the nasal cavities.

### Description of the Prior Art

It has been indicated that symptoms of nasal obstruction, apnea and snoring are caused by nasal septal deviation, chronic nasal inflammation, sinusitis, allergic nasal catarrh, adenoid enlargement, etc., the causes of which are as follows:

Nasal turbinates of nasal cavities, the inner parts of which are covered with mucosa, are full of veniplex. If the veniplex is swelled because of cold air or chilly atmosphere, the nasal cavities become narrow, and therefore, the amount of air drawn in through the nose becomes reduced, thereby causing troubles in respiration. If a person lies on a bed with his/her face directed upwardly during sleep under the condition that the nasal cavities are narrowed, the lingual root sags down inside the throat, causing an air passage to be narrowed or closed. And, if the person inhales through the narrowed passage, the relaxed soft palate is vibrated, which causes snoring.

If apnea or heavy snoring occurs during sleep, it is difficult for the person to satisfactorily breathe in air and it is not possible to experience a good sleep. Thus, he/she is still likely to feel sleepy after awakening and does not feel refreshed. If the person continuously suffers such conditions, his/her powers of concentration and judgment in daily life will decline. In addition, because the person becomes short of oxygen, this causes a strain on the heart, thereby contributing to heart disease because of hypertension and irregular pulse, etc.

To solve such problems, "a respiration-aiding device" has been proposed and a patent therefor was registered under Korean Patent No. 229700. This device is formed by an integral connection of portions to press the nasal septum. Both ends of a connection part are expanded with the fingers so as to widen the space between the pressing parts. The user inserts the widened pressing parts into the nostrils and then removes the fingers from the connection part. Then, the widened pressing parts become narrowed, and the nasal septum (the piece of bone that separates the nasal cavity into right and left sides) is pressed. If the nasal septum is applied with pressure, such a stimulus is transmitted to the brain which then controls the nose. The nasal meatus is widened in compliance with the stimulus, thereby facilitating passage of the air.

The conventional respiration-aiding device has been designed to allow the pressing parts elastically supported by the U-shaped connection part to be biased inwardly. If a person uses this device, the pressing parts apply pressure to both sides of the nasal septum. Thus, if the respiration-aiding device has been used repeatedly, and accordingly nerves of the nasal septum are pressed repeatedly, the nerves of the nasal septum become numb and the operational effect of the device is reduced gradually. In addition, since the device, as a result of repeated use, does not press both sides of the nasal septum with uniform pressure, a situation that the nasal septum is curved irregularly is caused. Such irregular curving of the nasal septum causes nasal obstruction and troubles in normal function of the nose, thereby leading to snoring during sleep.

### SUMMARY OF THE INVENTION

Accordingly, the present invention has been made keeping in mind the above-described problems of the conventional respiration-aiding device, and an object of the present invention is to provide an improved device for preventing nasal obstruction.

Another object of the present invention is to provide a device for preventing nasal obstruction, integrally combined with retainers, each of which has an air passage, on both sides of a U-shaped supporter so as to allow air to smoothly pass.

Still another object of the present invention is to provide a device for preventing nasal obstruction, in which if the retainers are inserted into the nasal cavities, nasal obstruction can be prevented even if the wall of the nasal meatus is strained, thereby facilitating respiration.

A further object of the invention is to provide a device for preventing nasal obstruction, wherein the retainers serve to maintain the nasal septum in a regular shape, thereby causing no deformation of the nasal septum or nasal blocking, even if the person wears the device for a long time, and preventing snoring during sleep.

In order to accomplish these and other objects of the present invention, there is provided a device for preventing nasal obstruction, comprising two pressing parts formed on both ends of a U-shaped supporter and used for pressing a nasal septum, and two retainers provided on both ends of the U-shaped supporter, in which a plurality of expansion-support plates and a plurality of air passages are alternately formed on each retainer.

### BRIEF DESCRIPTION OF THE DRAWINGS

The present invention will be better understood and the various objects, other features and advantages will be more fully understood from the following description in conjunction with the accompanying drawings, in which:
Fig. 1 is a perspective view showing a device for preventing nasal obstruction according to the primary embodiment of the present invention;
Figs. 2a and 2b are a front view and a side view of the device for preventing the nasal blocking shown in Fig. 1; and
Figs. 3a and 3b are views illustrating retainers of the device for preventing nasal obstruction according to the second and third embodiments of the present invention.

### DETAILED DESCRIPTION OF THE PREFERRED EMBODIMENTS

The preferred embodiments of the present invention will be described with reference to the accompanying drawings.

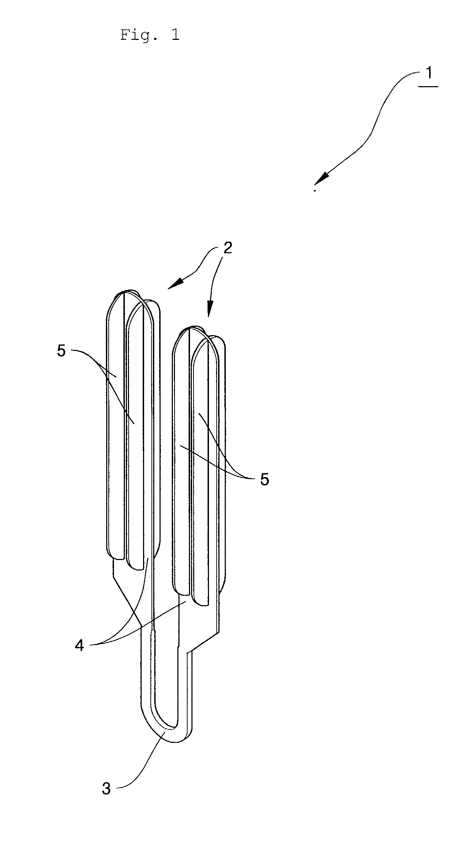

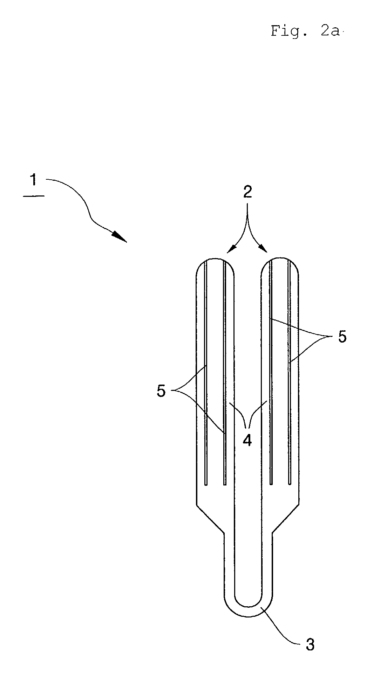

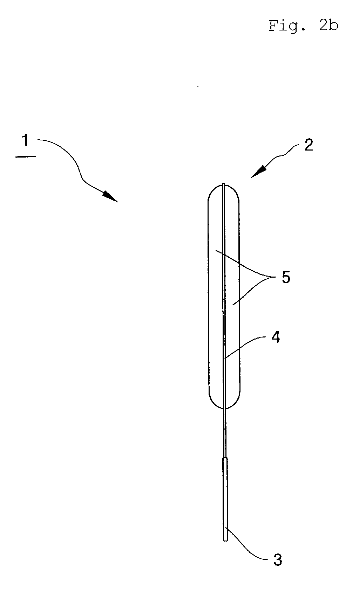

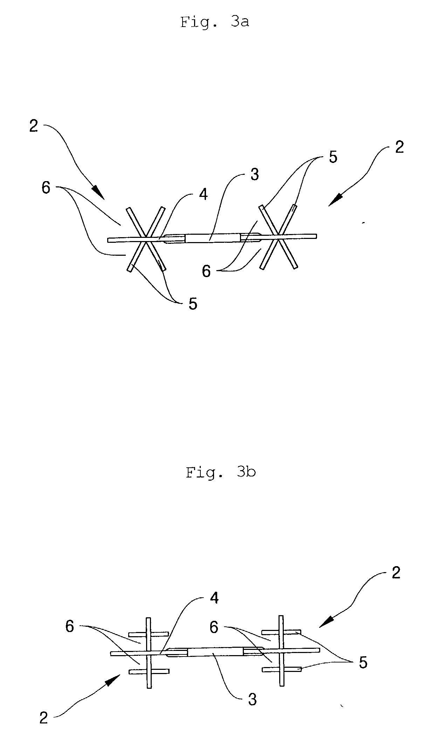

Figs. 1 through 3b illustrate the device 1 for preventing nasal obstruction according to the different embodiments of the present invention. Briefly, Fig. 1 is a perspective view showing the device for preventing nasal obstruction according to the primary embodiment, and Figs. 2a and 2b are a front view and a side view of the device of Fig. 1. Figs. 3a and 3b are top plan views illustrating the retainers of the device according to the second and third embodiment.

As shown in Figs. 1 through 2b, the device for preventing nasal obstruction according to the primary embodiment of the present invention is comprised of a U-shaped supporter 3 and two retainers 2 formed on both ends of the U-shaped supporter 3, wherein the supporter 3 and the retainers 2 are integrally combined with each other. In the retainers 2 a plurality of expansion-support plates 5 are integrally and in parallel formed on both sides of a main support plate 4, thereby forming a plurality of air passages 6 between the support plates 4 and expansion support plates 5. Upper and lower corners of the main support plates 4 and expansion-support plates 5 are rounded so as to enable them to be smoothly inserted into and removed from the nose without injuring the nose or causing pain to the user.

Figs. 3a and 3b are views illustrating second and third embodiments of the retainers 2. That is, Fig. 3a illustrates a retainer 2 having main support plates 4 and expansion-support plates 5 formed on each main support plate 4 while being spaced apart from each other and from the main support plate 4 at equal angles, thereby forming a plurality of air passages 6 between the main support plates 4 and the expansion-support plates 5.

In the retainer 2 depicted in Fig. 3b, the expansion-support plates 5 are formed in the shape of a cross at both sides of each main support plate 4, thereby forming a plurality of air passages 6 between the main support plates 4 and the expansion-support plates 5.

Hereinbelow, use of the device for preventing nasal obstruction according to the present invention is described in detail.

To use the nasal obstruction-preventing device 1, the U-shaped supporter 3 is held by a hand. Leading edges of the retainers 2 are positioned at the entrance of the nasal cavities and the U-shaped supporter 3 is pushed upward, thereby inserting the retainers 2 into the nasal cavities.

If the retainers 2 are inserted into the nasal cavities, the expansion-support plates 5 are situated so as to expand the walls of the nasal cavities. Therefore, the expansion-support plates 5 of the retainers 2 serve to prevent the walls of the nasal cavities from being strained even if the walls of the nasal cavities are strained, thereby preventing nasal obstruction. Therefore, this device facilitates respiration through the nose.

As illustrated in Figs. 1 through 2b, in the case of the retainers 2 each formed with expansion-support plates 5 on both sides of the main support plate 4, the walls of the nasal cavities are supported so as not to be contracted, owing to the main support plates 4 and the expansion-support plates 5. Sufficient air passages 6 are thus formed between the main support plates 4 and the expansion-support plates 5, thereby making it easy to respire through the air passages 6.

As depicted in Fig. 3a, in the case of the retainers 2 each having the expansion-support plates 5 expanded in all directions from the center, the walls of the nasal cavities remain expanded by the outer faces of the expansion-support plates 5, so respiration is facilitated through the air passages 6 formed between the main support plates 4 and the expansion-support plates 5.

As shown in Fig. 3b, in the case of the retainers 2 having the expansion-support plates 5 formed in the shape of a cross on both sides of each main support plate 4, the wall of the nasal cavity is supported by the main support plate 4 and the outer faces of the expansion-support plates 5, and the air passages 6 are formed between the plates 4 and 5. Therefore, respiration is facilitated through the air passages 6 secured between the main support plates 4 and the expansion-support plates 5.

As described above, the nasal obstruction-preventing device according to the present invention serves, by using the main support plates and the expansion-support plates, to prevent the walls of the nasal cavities from being undesirably strained, simply by inserting two retainers integrally formed on both sides of a U-shaped supporter into the nasal cavities. The device thus provides smooth respiration through the air passages formed between the main support plates and the expansion-support plates, and prevents apnea or snoring due to nasal obstruction during sleep, and further provides good sleep and preventing occurrence of heart disease due to insufficient supply of oxygen.

After use, the nasal obstruction-preventing device of this invention is removed from the nasal cavities, cleaned, rinsed, dried and disinfected, and then continues to be used. In addition, this nasal obstruction-preventing device is small in volume and easily carried in the hand. This device is also simple in structure, thereby causing no damage to or injury of the walls of the nasal cavities and facilitating use thereof.

Although the preferred embodiments of the present invention have been disclosed for illustrative purposes, those skilled in the art will appreciate that various modifications, additions and substitutions are possible, without departing from the scope and spirit of the invention as disclosed in the accompanying drawings.

## Claims

1. A device for preventing nasal obstruction, having a pressing part formed at each end of a U-shaped supporter, comprising:
a retainer provided on each end of said U-shaped supporter, with a plurality of expansion-support plates and a plurality of air passages alternately formed on said spacer retainer.

2. The device according to claim 1, wherein said retainer is comprised of the expansion-support plates integrally and in parallel formed on both sides of a main support plate, thereby forming the air passages between the main support plate and the expansion-support plates.

3. The device according to claim 1, wherein said retainer is comprised of a main support plate and the expansion-support plates formed on the main support plate while being spaced apart from each other and from the main support plate at equal intervals, thus forming the air passages between the main support plate and the expansion-support plates.

4. The device according to claim 1, wherein the retainer is comprised of the expansion-support plates in the form of a cross on both sides of a main support plate, thus forming the air passages between the main support plate and the expansion-support plates.
